# FASCICULE DE BREVET EUROPEEN

(11) **EP 0 982 305 B1**
(45) Date de publication et mention de la délivrance du brevet: **06.03.2002**
(21) Numéro de dépôt: 99402092.3
(22) Date de dépôt: 20.08.1999
(51) Int. Cl.: C07D 405/04, A61K 31/35

(54) **Nouveaux dérivés de l'indane-1-ol, leur procédé de préparation et les compositions pharmaceutiques qui les contiennent.**
Indane-1-ol Derivate, Verfahren zu deren Herstellung und diese enthaltende pharmazeutische Zusammenstellungen
Indane-1-ol derivatives, process for their preparation and pharmaceutical compositions containing them

(30) Priorité: 21.08.1998 FR 9810601
(43) Date de publication de la demande: 01.03.2000
(73) Titulaire: LES LABORATOIRES SERVIER, 92200 Neuilly sur Seine (FR)
(72) Inventeur: Peglion, Jean-Louis, 78110 Le Vesinet (FR); Goument, Bertrand, 78220 Viroflay (FR); Millan, Mark, 78230 Le Pecq (FR); Newman-Tancredi, Adrian, 78230 Le Pecq (FR)

(56) Documents cités:
- EP-A- 0 867 436
- WO-A-95/07274

## Description

La présente invention concerne de nouveaux dérivés de l'indane-1-ol, leur procédé de préparation et les compositions pharmaceutiques qui les contiennent.

Les composés de la présente invention, outre le fait qu'ils soient nouveaux, présentent une activité sérotoninergique particulièrement intéressante. Ainsi, les produits de la présente invention peuvent être utilisés comme médicaments dans le traitement de maladies dans lesquelles a été démontrée l'implication du système sérotoninergique telles que les maladies psychiatriques (dépression, anxiété, attaque de panique, schizophrénie, agressivité, troubles impulsifs, troubles obsessionnels compulsifs), les maladies dégénératives (Parkinson, Alzheimer), la douleur, la migraine, les céphalées, les accidents vasculaires cérébraux, la boulimie, l'anorexie, l'abus de drogue et aussi dans les maladies cardio-vasculaires (angor instable), puisque à l'instar du système nerveux central, le système sérotoninergique est aussi présent dans les territoires cardio-vasculaires.
De nombreux récepteurs à la sérotonine ont été identifiés et récemment clonés. Ils ont été classés, sur la base de leur structure primaire et de leur mode de couplage avec les systèmes de transduction, en sept classes majeures 5HT₁ à 5HT₇ (Molecular Biology of 5HT *Receptors, Neuropharmacol.*, **1994,** 33, *275).* Ces classes sont elles-mêmes subdivisées en sous-types. Pour le récepteur 5HT₁, on connaît les sous-types 5HT_{1A}, 5HT_{1B} (anciennement 5HT_{1Dβ}) et 5HT_{1D} (anciennement 5HT_{1Dα}) (pour une revue récente et une discussion de la nomenclature, *Trends in Pharmacol. Sciences,* **1996,** 17, 103).

L'application de la présente invention concerne plus particulièrement le récepteur 5HT_{1B} car les produits de l'invention se comportent comme des ligands puissants et sélectifs de ce récepteur. Les récepteurs 5HT_{1B} sont localisés post-synaptiquement au niveau cérébral ainsi que sur les terminaisons nerveuses sympathiques périphériques, les vaisseaux sanguins cérébraux, les afférences primaires trigéminales (*Naunyn-Schmiedeberg's Arch. Pharmacol.*, **1990,** 342, 371 ; *Mol. Pharmacol.*, **1993,** 44, 242 ; *Eur. J. Pharmacolo.*, **1992,** 227, 357). Cette localisation implique que, par activation des populations de récepteurs 5HT_{1B}, par un effet aussi bien vasculaire que neurogénique il est possible de soigner avec des agonistes les migraines et les céphalées. Avec des antagonistes, par action sur les récepteurs périphériques il sera possible de soigner des maladies du système cardio-vasculaire telles que l'angor instable. D'autre part ces populations de récepteurs 5HT_{1B}, qui sont aussi présentes en fortes concentrations dans la corne dorsale de la moelle épinière, le ganglion basal, l'hippocampe et les autres structures limbiques du cortex frontal (*Naunyn-Schmiedeberg's Arch. Pharmacol.*, **1992,** 347, 248 ; *Eur. J. Pharmacol.*, **1992,** 222, 137 ; *J. Neurochem.*, **1995,** 65, 2671), peuvent être en partie responsables des troubles de l'humeur et du comportement et être impliqués dans les mécanismes de nociception. Du fait d'une double localisation, d'une part sur les neurones sérotoninergiques post-synaptiques, et d'autre part sur les corps cellulaires où ils jouent le rôle d'autorécepteurs, on peut facilement conclure à leur implication dans la pathogénèse, et par voie de conséquence en utilisant des ligands sélectifs de ces récepteurs, dans les traitements de la dépression, l'anxiété, les troubles impulsifs et les autres maladies psychiatriques liées à un dysfonctionnement de la transmission sérotoninergique (*Neurochem. Res.,* **1990,** 15, 567 ; *J. Neurochem.*, **1988,** 51, 1906).

En ce qui concerne le récepteur 5HT_{1B} (ex 5HT_{1Dβ}) il est prédominant dans le système nerveux central de l'homme et du cobaye. De plus, seuls les récepteurs 5HT_{1B} sont localisés comme autorécepteurs, ce qui n'est pas le cas des récepteurs 5HT_{1D} (ex 5HT_{1Dα}). Des ligands des récepteurs 5HT_{1B}/5HT_{1D} ont été décrits dans les demandes WO 96/00720 et WO 96/12713 : il s'agit de dérivés naphthylpipérazine. Des antagonistes des récepteurs 5HT_{1B}/5HT_{1D} de structures biphényliques ont été décrits dans la demande WO 96/19477. Ces structures ne suggèrent en rien les composés de la présente invention. La demande de brevet WO 95/07274 fait état de composés utilisés dans le traitement des maladies du système nerveux central qui possèdent une structure 4-amino pipéridine. Dans la formule générale de cesdits composés, l'azote extracyclique est relié, par l'intermédiaire d'une chaîne alkane, à des noyaux benzodioxane, tétrahydronaphtalène et chromane. Ces structures n'induisent donc nullement celles de la présente invention.

Plus spécifiquement, la présente invention concerne les composés de formule (I) : 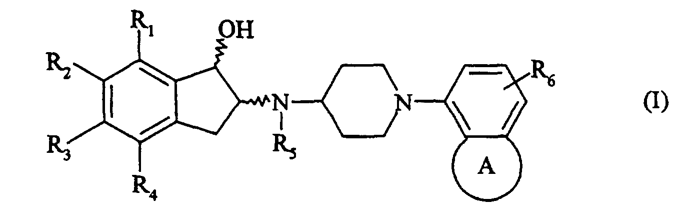 dans laquelle :
- R₁, R₂, R₃, R₄, identiques ou différents, indépendamment l'un de l'autre, représentent un atome d'hydrogène, d'halogène, un groupement alkyle (C₁-C₆) linéaire ou ramifié, alkényle (C₂-C₆) linéaire ou ramifié, alkynyle (C₂-C₆) linéaire ou ramifié, hydroxy, alkoxy (C₁-C₆) linéaire ou ramifié, trihalogénoalkyle (C₁-C₆) linéaire ou ramifié, ou lorsqu'ils sont pris deux à deux, en position adjacente, forment un hétérocycle avec les atomes de carbone du noyau phényle auxquels ils sont liés,
- R₅ représente un atome d'hydrogène, un groupement alkyle (C₁-C₆) linéaire ou ramifié, ou arylalkyle (C₁-C₆) linéaire ou ramifié,
- R₆ représente un atome d'hydrogène, d'halogène, un groupement alkyle (C₁-C₆) linéaire ou ramifié, hydroxy, alkoxy (C₁-C₆) linéaire ou ramifié, ou un groupement trihalogénoalkyle (C₁-C₆) linéaire ou ramifié,
- A représente un hétérocycle avec les atomes de carbone du noyau phényle auxquels ils sont liés,
leurs isomères ainsi que leur sels d'addition à un acide ou à une base pharmaceutiquement acceptable.

Etant entendu que par hétérocycle, on comprend un groupement monocyclique, de 5 à 7 chaînons, comportant éventuellement une ou deux insaturations supplémentaires que celle due à la jonction dudit hétérocycle avec le noyau phényle auquel il est fusionné, et contenant un, deux ou trois hétéroatomes, identiques ou différents, choisis parmi oxygène, soufre ou azote. Par groupement aryle, on comprend un groupement phényle, naphtyle, dihydronaphtyle ou tétrahydronaphtyle.

Selon une variante avantageuse de l'invention, les composés préférés sont ceux pour lesquels A représente, avec les atomes de carbone du noyau phényle auxquels ils ont liés, un hétérocycle à 6 chaînons contenant au moins un atome d'oxygène. D'une façon préférentielle A représente, avec le noyau phényle auquel il est lié, un groupement chromane ou 2,3-dihydro-1,4-benzodioxine.

Le substituant R₅ préféré selon l'invention est l'atome d'hydrogène.

D'une façon avantageuse, les composés préférés de l'invention sont ceux pour lesquels R₁, R₂, R₃ et R₄, identiques ou différents, indépendamment l'un de l'autre, représentent un atome d'hydrogène, d'halogène, un groupement alkyle (C₁-C₆) linéaire ou ramifié, ou lorsqu'ils sont pris deux à deux, en positions adjacentes, forment, avec les atomes de carbone du noyau phényle auxquels ils sont liés, un hétérocycle à 5 chaînons contenant un ou deux atomes d'oxygène.

Le substituant R₆ préféré selon l'invention est l'atome d'hydrogène ou d'halogène.

D'une façon particulièrement avantageuse, les composés préférés de l'invention sont :
- le 2-{[1-(3,4-dihydro-2*H*-chromén-8-yl)-4-pipéridyl]amino}-5-fluoro-1-indanol,
- le 2-{[1-(2,3-dihydro-1,4-benzodioxin-5-yl)-4-pipéridyl]amino}-1-indanol,
- le 2-{[1-(3,4-dihydro-2*H*-chromén-8-yl)-4-pipéridyl]amino}-6-fluoro-1-indanol,
- le 2-{[1-(2,3-dihydro-1,4-benzodioxin-5-yl)-4-pipéridyl]amino}-6-méthyl-1-indanol,
- le 2-{[1-(3,4-dihydro-2*H*-chromén-8-yl)-4-pipéridyl]amino}-5,6-méthylènedioxy-1-indanol,
- et le 2-{[1-(3,4-dihydro-2*H*-chromén-8-yl)-4-pipéridyl]amino}-5,6-dihydrofuro[2,3-*b*]indan-1-ol.

Les isomères ainsi que les sels d'addition à un acide ou à une base pharmaceutiquement acceptable, des composés préférés font partie intégrante de l'invention.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I), caractérisé en ce que l'on utilise comme produit de départ, un composé de formule (II) : 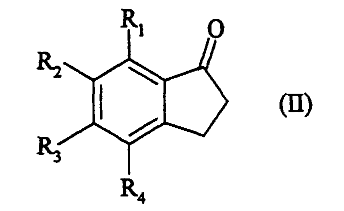 dans laquelle R₁, R₂, R₃ et R₄ ont la même signification que dans la formule (I),
composés de formule (II) que l'on fait réagir avec de l'isoamylnitrite, en présence d'acide chlorhydrique dans le méthanol, pour conduire aux composés de formule (III) : 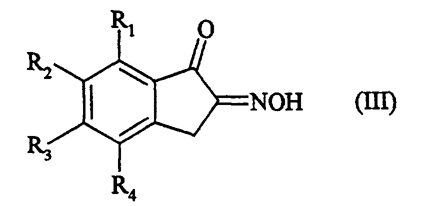 dans laquelle R₁, R₂, R₃ et R₄ sont tels que définis précédemment,
composés de formule (III) que l'on place en présence d'un agent de réduction, pour conduire aux composés de formule (IV), 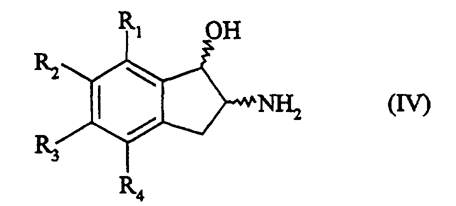 dans laquelle R₁, R₂, R₃ et R₄ sont tels que définis précédemment,
composés de formule (IV) que l'on traite, dans des conditions d'amination réductrice, par un composé de formule (V) : 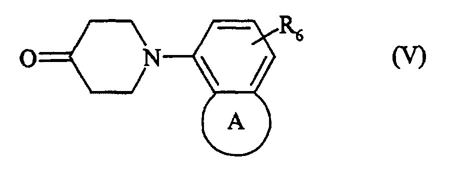 dans laquelle A et R₆ ont la même signification que dans la formule (I),
pour conduire aux composés de formule (I/a), cas particulier des composés de formule (I) : 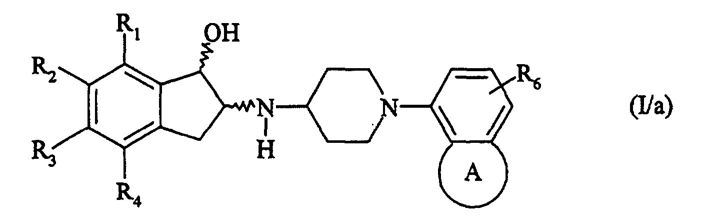 dans laquelle R₁, R₂, R₃, R₄, R₆ et A sont tels que définis précédemment,
composés de formule (I/a) que l'on sépare selon des méthodes classiques de séparation (distillation, recristallisation, chromatographie), en ces deux composants de formules (I/a *cis*) et (I/a *trans*), cas particuliers des composés de formule (I) : 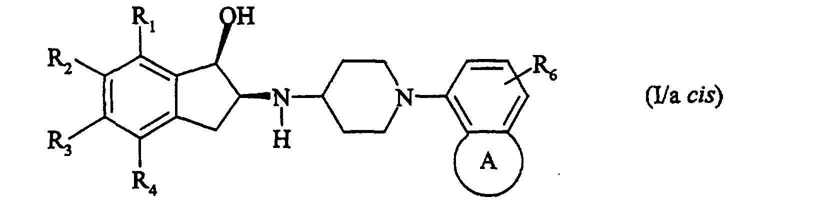 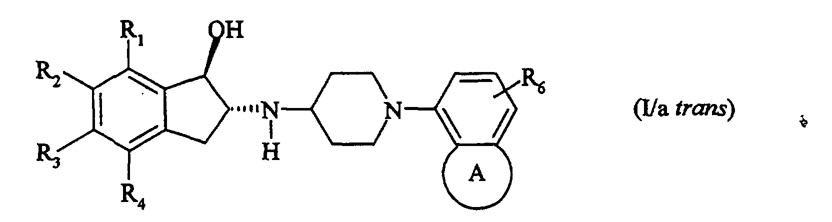 dans lesquelles R₁, R₂, R₃, R₄, R₆ et A sont tels que définis précédemment,
composés de formules (I/a), (I/a *cis*) et (I/a *trans*), dont on substitue, si on le désire, la fonction amine secondaire, selon des méthodes classiques de la chimie organique, en utilisant un composé de formule (VI) :

R'₅-Z (VI)

dans laquelle R'₅ représente un groupement alkyle (C₁-C₆) linéaire ou ramifié ou arylalkyle (C₁-C₆) linéaire ou ramifié, et Z un groupement libérable,
pour conduire respectivement aux composés de formules (I/b), (I/b *cis*) et (I/b *trans*) : 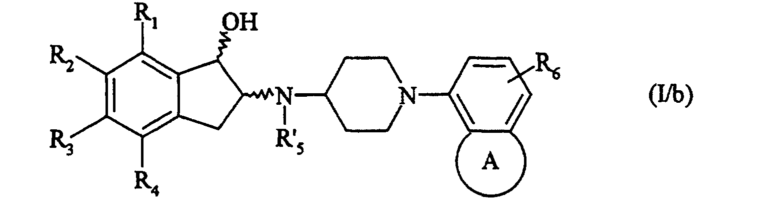 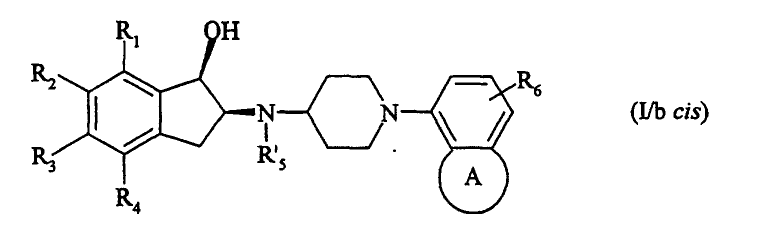 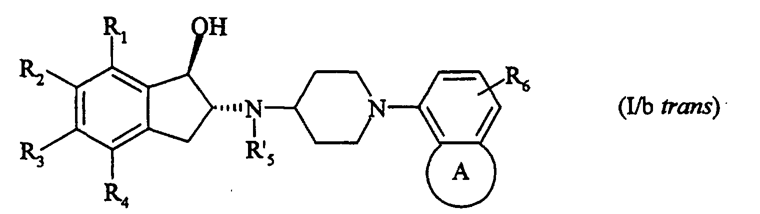 dans lesquelles R₁, R₂, R₃, R₄, R'₅, R₆ et A sont tels que définis précédemment,
les composés de formules (I/a), (I/b), (I/a *cis),* (I/a *trans* ), (I/b *cis)* et (I/b *trans*), formant l'ensemble des composés de formule (I), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les composés de formule (II), (V) et (VI) sont soit des composés commerciaux, soit obtenus selon des méthodes classiques de la synthèse organique.

Les composés de formule (I) présentent une excellente affinité sélective pour les récepteurs sérotoninergiques 5HT_{1B}. Cette sélectivité pour les récepteurs 5HT_{1B} a pu être évaluée au cours d'expérience de binding notamment vis à vis des récepteurs 5HT_{1A}. Les composés de la présente invention sont donc utiles pour le traitement des maladies dans lesquelles les récepteurs 5HT_{1B} interviennent.

La présente invention a également pour objet les compositions pharmaceutiques renfermant comme principe actif au moins un composé de formule (I), ses isomères ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou trans-cutanée, nasale, rectale, perlinguale, oculaire ou respiratoire, et notamment les comprimés simples ou dragéifiés, les comprimés sublinguaux, les sachets, les gélules, les capsules, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 0,5 à 25 mg de principe actif en une ou plusieurs prises par jour.

Les exemples suivants illustrent l'invention mais ne la limitent en aucune façon.

Les produits de départ utilisés sont soit des produits connus ou préparés selon des modes opératoires connus.

Les différentes préparations conduisent à des intermédiaires de synthèse utiles pour la préparation des composés de l'invention.

Les structures des composés décrits dans les exemples et les préparations ont été déterminées selon les techniques spectrophotométriques usuelles (infrarouge, résonance magnétique nucléaire, spectromètre de masse...). Les points de fusion ont été déterminés soit à la platine chauffante de Kofler (K.), soit à la platine chauffante sous microscope (M.K.).

### Préparation 1 : 1-(Chroman-8-yl)piperidin-4-one

### Stade 1 : 8-Nitro-2H-chromène

Une solution de 20 g de 8-nitro-4-chromanone et 3,9 g de borohydrure de sodium dans 200 ml d'éthanol, est chauffée au reflux pendant 45 minutes puis évaporée à sec. Le résidu est repris par 200 ml d'eau, extrait par trois fois avec 250 ml de dichlorométhane. Les phases organiques rassemblées sont séchées sur sulfate de magnésium, filtrées puis concentrées sous pression réduite. Le résidu liquide est repris par 300 ml de toluène, puis 0,5 g d'acide *para*-toluène sulfonique est additionné et on chauffe à reflux jusqu'à ce que toute la quantité d'eau attendue ait été récupérée. Après refroidissement, lavage par une solution à 5 % de carbonate de sodium puis à l'eau, et concentration sous pression réduite on isole un résidu liquide qui correspond au produit attendu .

### Stade 2 : 8-Amino-chromane

Le produit obtenu au stade précédent est hydrogéné en présence d'oxyde de platine dans le méthanol pour conduire après évaporation et passage acide-base au produit attendu.

### Stade 3 : 1-(Chroman-8-yl) piperidin-4-ol

Une solution de 6,3 g du produit obtenu au stade précédent, 6,6 g de 1,5-dichloro-pentan-3-ol, 11,65 g de carbonate de potassium et 3,16g d'iodure de sodium dans 23 ml de diméthylformamide est chauffée pendant 1,5 heures. Après retour à température ambiante 230 ml d'eau sont ajoutés puis la solution est extraite à l'éther. Après séchage de la phase organique sur sulfate de magnésium, filtration et concentration sous pression réduite, on obtient une huile qui correspond au produit attendu.

### Stade 4 : 1-(Chroman-8-yl)piperidin-4-one

A une solution de 8,5 g du produit obtenu au stade précédent, 29,9 g de dicyclohexylcarbodiimide, 5,4 ml de pyridine et 115 ml de diméthylsulfoxyde, dans 207 ml de toluène, refroidie à 5°C, sont additionnés, goutte à goutte, 3,6 ml d'acide trifluoroacétique. Après 24 heures de réaction à température ambiante, 400 ml d'acétate d'éthyle sont ajoutés, puis la solution est filtrée. Le filtrat est alors lavé à l'eau puis par une solution saturée en chlorure de sodium, séché sur sulfate de magnésium, puis concentré sous pression réduite. Une chromatographie sur gel de silice (dichlorométhane/acétate d'éthyle : 95/5) permet d'isoler le produit attendu.

### Préparation 2 : 1-(2,3-Dihydro[1,4]benzodioxin-5-yl)piperidin-4-one

On procède comme dans la préparation 1, en commençant au stade 3, et en utilisant comme substrat dans ce stade 3, la 5-amino-2,3-dihydro[1,4]benzodioxine.

### Préparation 3 : 1-(7-Chloro-2,3-dihydro[1,4]benzodioxin-5-yl)-pipéridine-4-one

On procède comme dans la préparation 1, en commençant au stade 3, et en utilisant comme substrat dans ce stade 3 la 5-amino-7-chloro-2,3-dihydro[1,4]benzodioxine.

### EXEMPLE 1 : (cis)-2-{[1-(3,4-Dihydro-2H-chromén-8-yl)-4-pipéridyl]amino}-5-fluoro-1-indanol

### Stade A : 5-Fluoro-2-hydroxyimino-1-indanone

A une solution de 11,2 g de 5-fluoro-1-indanone dans 185 ml de méthanol portée à 40°C sont additionnés goutte à goutte 15 ml d'isoamylnitrite, puis 7,5 ml d'acide chlorhydrique concentré. Après 45 minutes de réaction à 40°C, le milieu réactionnel est refroidi par de la glace. Il y a formation d'un précipité qui est filtré puis séché sous vide permettant d'isoler le produit attendu.

### Stade B : 2-Amino-5-fluoro-1-indanol (mélange cis/trans)

A une suspension de 2,67g d'hydrure de lithium et d'aluminium dans 15 ml de tétrahydrofurane, est additionné goutte à goutte en 30 minutes le produit obtenu au stade A dissous dans 70 ml de tétrahydrofurane. Après 30 minutes d'agitation à température ambiante, puis 12 heures au reflux du solvant, le milieu réactionnel est refroidi puis hydrolysé successivement avec précaution par 1,9 ml d'eau, 1,5 ml de soude à 20 %, puis 6,7 ml d'eau. La solution est alors agitée 3 heures à température ambiante, puis filtrée, rincée au tétrahydrofurane et évaporée à sec. Une chromatographie sur gel de silice (dichlorométhane/méthanol/hydroxyde d'ammonium : 95/5/0,5) permet d'isoler le produit attendu dans des proportions de 54% de produit *cis* et 46% de produit *trans*.

### Stade C : (cis)-2-{[1-(3,4-Dihydro-2H-chromén-8-yl)-4-pipéridyl]amino}-5-fluoro-1-indanol

A 0,92 g du mélange obtenu dans le stade B et 1,27 g du produit obtenu dans la préparation 1, dissous dans 40 ml de 1,2-dichloroéthane sont additionnés rapidement 1,63 g de triacétoxyborohydrure de sodium et 0,31 ml d'acide acétique. Après 48 heures de réaction à température ambiante, le milieu réactionnel est versé sur 40 ml de soude 1N puis extrait par 80 ml d'éther. Après filtration de l'insoluble, la phase aqueuse est à nouveau extraite à l'éther. Les phases organiques réunies sont séchées sur sulfate de magnésium, filtrées puis concentrées sous pression réduite. Une chromatographie sur gel de silice (dichlorométhane/méthanol : 96/4), suivie d'une recristallisation de l'acétonitrile permet d'isoler le produit attendu.
*Point de fusion : 172-174°C (M.K.)*

| *Microanalyse élémentaire :* | | | |
|---|---|---|---|
| | *%C* | *%H* | *%N* |
| *calculé* | *72,23* | *7,11* | *7,32* |
| *trouvé* | *72,51* | *7,24* | *7,32* |

### EXEMPLE 2 : (trans)-2-{[1-(3,4-Dihydro-2H-chromén-8-yl)-4-pipéridyl]amino}-5-fluoro-1-indanol

L'insoluble obtenu dans le stade C de l'exemple 1 est recristallisé deux fois de l'acétonitrile, permettant d'isoler le produit attendu.
*Point de fusion : 176-178°C (M.K.)*

| *Microanalyse élémentaire :* | | | |
|---|---|---|---|
| | *% C* | *% H* | *% N* |
| *calculé* | *72, 23* | *7,11* | *7,32* |
| *trouvé* | *72,34* | *7,23* | *7,36* |

### EXEMPLE 3 : (cis)-2-{[1-(2,3-Dihydro-1,4-benzodioxin-5-yl)-4-pipéridyl] amino}-1-indanol

On procède comme dans l'exemple 1, des stades A à C, en utilisant comme substrat au stade A la 1-indanone, et au stade C, le produit obtenu dans la préparation 2 à la place du produit obtenu dans la préparation 1.
*Point de fusion : 178-180°C (M.K.)*

| *Microanalyse élémentaire :* | | | |
|---|---|---|---|
| | % *C* | *% H* | % *N* |
| *calculé* | *72,11* | *7,*15 | *7,64* |
| *trouvé* | *72,00* | *7,03* | *7,63* |

### EXEMPLE 4 : (trans)-2-{[1-(2,3-Dihydro-1,4-benzodioxin-5-yl)-4-pipéridyl] amino}-1-indanol

On procède comme dans l'exemple 2 à partir de l'insoluble obtenu dans le stade C de l'exemple 3.
*Point de fusion : 199-201°C(M.K.)*

| *Microanalyse élémentaire :* | | | |
|---|---|---|---|
| | *% C* | *% H* | *% N* |
| *calculé* | *72,11* | *7*,*15* | *7,64* |
| *trouvé* | *71,79* | *7,59* | *7,49* |

### EXEMPLE 5 : (cis)-2-{[1-(7-Chloro-2,3-dihydro-1,4-benzodioxin-5-yl)-4-pipéridyl]amino}-6-fluoro-1-indanol

On procède comme dans l'exemple 1 des stades A à C en utilisant comme substrat au stade A, la 6-fluoro-1-indanone et au stade C, le produit obtenu dans la préparation 3 à la place du produit obtenu dans la préparation 1.
***Stade A : 6-Fluoro-2-hydroxyimino-1-indanone***
***Stade B : 2-Amino-6-fluoro-1-indanol (mélange cis/trans)***
***Stade C : (cis)-2-{[1-(7-Chloro-2,3-dihydro-1,4-benzodioxin-5-yl)-4-pipéridyl]amino}-6-fluoro-1-indanol***
*Point de fusion : 184-186°C (M.K.)*

### EXEMPLE 6: (trans)-2-{[1-(7-Chloro-2,3-dihydro-1,4-benzodioxin-5-yl)-4-pipéridyl]amino}-6-fluoro-1-indanol

On procède comme dans l'exemple 2 à partir de l'insoluble obtenu dans le stade C de l'exemple 5.
*Point de fusion : 193-197°C (M.K.)*

### EXEMPLE 7 : (cis)-2-{[1-(3,4-Dihydro-2H-chromén-8-yl)-4-pipéridyl]amino}-6-fluoro-1-indanol

On procède comme dans l'exemple 1, des stades A à C, en utilisant comme substrat au stade A le composé du stade A de l'exemple 5, et au stade C le composé obtenu au stade B de l'exemple 5.
*Point de fusion : 179-181°C (M.K.)*

### EXEMPLE 8: (trans)-2-{[1-(3,4-Dihydro-2H-chromén-8-yl)-4-pipéridyl]amino}-6-fluoro-1-indanol

On procède comme dans l'exemple 2 à partir de l'insoluble obtenu dans le stade C de l'exemple 7.
*Point de fusion : 199-204°C (M.K.)*

### EXEMPLE 9 : (cis)-2-{[1-(3,4-Dihydro-2H-chromén-8-yl)-4-pipéridyl]amino}-5,6-méthylènedioxy-1-indanol

On procède comme dans l'exemple 1 des stades A à C en utilisant comme substrat au stade A la 5,6-méthylènedioxy-1-indanone.
***Stade A : 2-Hydroxyimino-5,6-méthylènedioxy-1-indanone***
***Stade B : 2-Amino-5,6-méthylènedioxy-1-indanol (mélange cis/trans)***
***Stade C : (cis)-2-{[1-(3,4-Dihydro-2H-chromén-8-yl)-4-pipéridyl]amino}-5,6-méthylènedioxy-1-indanol***
*Point de fusion : 180-183°C (M.K.)*

### EXEMPLE 10 : (trans)-2-{[1-(3,4-Dihydro-2H-chromén-8-yl)-4-pipéridyl]amino}-5,6-méthylènedixoy-1-indanol

On procède comme dans l'exemple 2 à partir de l'insoluble obtenu au stade C de l'exemple 9.
*Point de fusion : 179-181°C (M.K.)*

### EXEMPLE 11 : (cis)-2-{[1-(3,4-Dihydro-2H-chromén-8-yl)-4-pipéridyl]amino}-5,6-dihydrofuro[2,3-b]indan-1-ol

On procède comme dans l'exemple 1, des stades A à C, en utilisant comme substrat au stade A, la 5,6-dihydrofuro[2,3-*b*]indan-1-one.
***Stade A : 2-Hydroxyimino-5,6-dihydrofuro[2,3-b]indan-1-one***
***Stade B : 2-Amino-5,6-dihydrofuro[2,3-b]indan-1-ol (mélange cis/trans)***
***Stade C : (cis)-2-{[1-(3,4-Dihydro-2H-chromén-8-yl)-4-pipéridyl]amino}-5,6-dihydrofuro[2,3-b]indan-1-ol***
*Point de fusion : 201-203°C (M.K.)*

### EXEMPLE 12 : (trans)-2-{[1-(3,4-Dihydro-2H-chromén-8-yl)-4-pipéridyl]amino}-5,6-dihydrofuro[2,3-b]indan-1-ol

On procède comme dans l'exemple 2 à partir de l'insoluble obtenu au stade C de l'exemple 11.
*Point de fusion : 193-195°C (M.K.)*

### EXEMPLE 13 : (cis)-2-{[1-(3,4-Dihydro-2H-chromén-8-yl)-4-pipéridyl]amino}-6,7-diméthyl-1-indanol

On procède comme dans l'exemple 1, des stades A à C, en utilisant comme substrat au stade A, la 6,7-diméthyl-1-indanone.
***Stade A : 6,7-Diméthyl-2-hydroxyimino-1-indanone***
***Stade B : 2-Amino-6,7-diméthyl-1-indanol (mélange cis/trans)***
***Stade C : (cis)-2-{[1-(3,4-Dihydro-2H-chromén-8-yl)-4-pipéridyl]amino}-6,7-diméthyl-1-indanol***
*Point de fusion : 158-160°C (M.K.)*

### EXEMPLE 14 : (cis)-2-{[1-(3,4-Dihydro-2H-chromén-8-yl)-4-pipéridyl]amino}-4,7-diméthyl-1-indanone

On procède comme dans l'exemple 1, des stades A à C, en utilisant comme substrat au stade A, la 4,7-diméthyl-1-indanol.
***Stade A : 4,7-Diméthyl-2-hydroxyimino-1-indanone***
***Stade B : 2-Amino-4,7-diméthyl-1-indanol (mélange cis/trans)***
***Stade C : (cis)-2-{[1-(3,4-Dihydro-2H-chromén-8-yl)-4-pipéridyl]amino}-4,7-diméthyl-1-indanol***
*Point de fusion : 169-172°C (M.K.)*

### EXEMPLE 15 : (trans)-2-{[1-(3,4-Dihydro-2H-chromén-8-yl)-4-pipéridyl]amino}-4,7-diméthyl-1-indanol

On procède comme dans l'exemple 2 à partir de l'insoluble obtenu au stade C de l'exemple 14.
*Point de fusion : 154-156°C (M.K.)*

### EXEMPLE 16 : (cis)-2-{[1-(2,3-Dihydro-1,4-benzodioxin-5-yl)-4-pipéridyl]amino}-6-méthyl-1-indanol

On procède comme dans l'exemple 1, des stades A à C, en utilisant comme substrat au stade A, la 6-méthyl-1-indanone, et au stade C, le produit obtenu dans la préparation 2 à la place du produit obtenu dans la préparation 1.
***Stade A : 2-Hydroxyimino-6-méhtyl-1-indanone***
***Stade B : 2-Amino-6-méthyl-1-indanol (mélange cis/trans)***
***Stade C : (cis)-2-{[1-(2,3-Dihydro-1,4-benzodioxin-5-yl)-4-pipéridyl]amino}-6-méthyl-1-indanol***
*Point de fusion : 169-170°C (M.K.)*

### EXEMPLE 17 : (trans)-2-{[1-(2,3-Dihydro-1,4-benzodioxin-5-yl)-4-pipéridyl]amino}-6-méthyl-1-indanol

On procède comme dans l'exemple 2 à partir de l'insoluble obtenu dans le stade C de l'exemple 16.
*Point de fusion : 203-205°C (M.K.)*

### ETUDE PHARMACOLOGIQUE DES COMPOSES DE L'INVENTION

### EXEMPLE 18 : Etudes de liaison

### • Liaison 5-HT_{1B}

### a) Préparation des membranes

Après dissection des cerveaux de cobaye, les striatums extraits sont congelés puis homogénéisés dans 20 volumes (poids/volume) de Tris-HCl 50 mM (pH 7,7 à température ambiante) contenant 4 mM de CaCl₂, 0,1 % d'acide ascorbique et enfin centrifugées à 48.000 g pendant 25 minutes à 4°C. Le surnageant est séparé et le précipité est remis en suspension dans le même volume de tampon avant d'être incubé à 37°C pendant 15 minutes pour retirer la sérotonine endogène. Finalement la suspension est centrifugée à 48.000 g pendant 25 minutes à 4°C et le précipité remis en suspension dans 80 volumes de tampon qui contient 10 µM de pargylline.

### b) Etude de liaison

Les études de liaison ([³H]-GR 125743) sont effectuées en triple dans le tampon suivant : 50 mM Tris-HCl (pH 7,7 à température ambiante) contenant 4 mM de CaCl₂, 0,1 % d'acide ascorbique et 10 µM de pargylline. Le volume final de 500 µl est constitué de 100 µl de radioligand, 100 µl de tampon ou de composé à tester et 300 µl de membranes. La sérotonine (10 µM) est utilisée pour définir la liaison non spécifique. Dans les expériences de compétition, la concentration de ([³H]-GR 125743) est de 1 nM. Les incubations sont initiées par l'addition de la préparation membranaire et durent 60 minutes à température ambiante. La réaction est stoppée par filtration rapide au travers de filtres pré-traités avec 0,1 % de polyéthylènimine, suivi par trois rinçages avec du tampon froid. La liaison spécifique représente environ 90 % de la liaison totale aux concentrations de radioligand proche de la valeur du Kd.

### • Analyses de données

Les données sont analysées par régression non linéaire pour déterminer les valeurs du Kd (constante de dissociation du radioligand) et du Bmax (nombre de sites maximum) pour les expériences de saturation, et celles de l'IC₅₀ (concentration inhibitrice à 50%) et du nombre de Hill pour les expériences de compétition. La constante d'inhibition (Kᵢ) est calculée selon l'équation de Cheng-Prussof : Kᵢ = IC₅₀ / 1 + L / K_{d}) dans laquelle L représente la concentration du radioligand. Les résultats sont exprimés en pKᵢ = - log Ki.
Les composés de la présente invention montrent tous une bonne affinité pour le récepteur 5-HT_{1B}. Leur pKᵢ est supérieur à 7,4.

### • Liaison 5-HT_{1A}

Les études de liaison sur le récepteur 5-HT_{1A} ont été effectuées selon les méthodes connues et décrites dans la littérature (*J. Neurochem.,* **1986,** 47, 529-40 ; *J. Pharmacol. Exp. Ther.*, **1994,** 268, 337-52). Les résultats sont aussi exprimés en pKᵢ.
Les composés de la présente invention sont peu affins pour le récepteur 5-HT_{1A}. Leur pKᵢ est de l'ordre de 6.

Ces deux études de liaison démontrent la sélectivité des produits de l'invention pour les récepteurs 5-HT_{1B} vis-à-vis des récepteurs 5-HT_{1A}.

### EXEMPLE 19 : Composition pharmaceutique : comprimés

Formule de préparation pour 1000 comprimés dosés à 5 mg
Composé de l'exemple 1 5 g
Hydroxypropylcellulose 2 g
Amidon de blé 10 g
Lactose 100 g
Stéarate de magnésium 2 g
Talc 2 g

## Revendications

1. Composés de formule (I) : 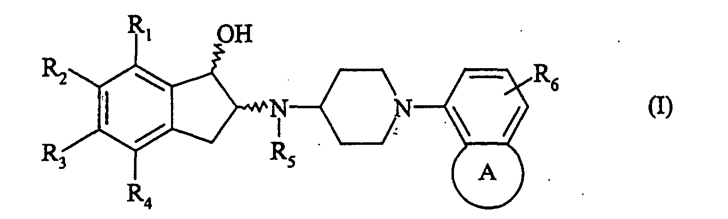 dans laquelle :
• R₁, R₂, R₃, R₄, identiques ou différents, indépendamment l'un de l'autre, représentent un atome d'hydrogène, d'halogène, un groupement alkyle (C₁-C₆) linéaire ou ramifié, alkényle (C₂-C₆) linéaire ou ramifié, alkynyle (C₂-C₆) linéaire ou ramifié, hydroxy, alkoxy (C₁-C₆) linéaire ou ramifié, trihalogénoalkyle (C₁-C₆) linéaire ou ramifié, ou lorsqu'ils sont pris deux à deux, en position adjacente, forment un hétérocycle constitué d'un groupement monocyclique, de 5 à 7 chaînons, comportant éventuellement une ou deux insaturations supplémentaires que celle due à la jonction dudit hétérocycle avec le noyau phényle auquel il est fusionné, et contenant un, deux ou trois hétéroatomes, identiques ou différents choisis parmi oxygène, soufre ou azote,
• R₅ représente un atome d'hydrogène, un groupement alkyle (C₁-C₆) linéaire ou ramifié, ou arylalkyle (C₁-C₆) linéaire ou ramifié,
• R₆ représente un atome d'hydrogène, d'halogène, un groupement alkyle (C₁-C₆) linéaire ou ramifié, hydroxy, alkoxy (C₁-C₆) linéaire ou ramifié, ou un groupement trihalogénoalkyle (C₁-C₆) linéaire ou ramifié,
• A représente, avec les atomes de carbone du noyau phényle auxquels ils sont liés, un groupement chromane ou 2,3-dihydro-1,4-benzodioxine,
leurs isomères ainsi que leur sels d'addition à un acide ou à une base pharmaceutiquement acceptable.

2. Composés de formule (I) selon la revendication 1 **caractérisés en ce que** R₅ représente un atome d'hydrogène, leurs isomères ainsi que leur sels d'addition à un acide ou à une base pharmaceutiquement acceptable.

3. Composés de formule (I) selon la revendication 1 **caractérisés en ce que** R₁, R₂, R₃ et R₄, identiques ou différents, indépendamment l'un de l'autre, représentent un atome d'hydrogène, d'halogène, un groupement alkyle (C₁-C₆) linéaire ou ramifié, ou lorsqu'ils sont pris deux à deux, en positions adjacentes, forment, avec les atomes de carbone du noyau phényle auxquels ils sont liés, un hétérocycle à 5 chaînons contenant un ou deux atomes d'oxygène, leurs isomères, ainsi que leurs sels d'addition à un acide ou à une base pharmaceutiquement acceptable.

4. Composés de formule (I) selon la revendication 1 **caractérisés en ce que** R₆ représente un atome d'hydrogène ou d'halogène, leurs isomères ainsi que leurs sels d'addition à un acide ou à une base pharmaceutiquement acceptable.

5. Composés de formule (I) selon la revendication 1 qui sont le (*cis*)-2-{[1-(3,4-dihydro-2*H*-chromén-8-yl)-4-pipéridyl]amino}-5-fluoro-1-indanol et le (*trans*)-2-{[1-(3,4-dihydro-2*H*-chromén-8-yl)-4-pipéridyl]amino}-5-fluoro-1-indanol, ainsi que leurs sels d'addition à un acide ou à une base pharmaceutiquement acceptable.

6. Composés de formule (I) selon la revendication 1 qui sont le (*cis*)-2-{[1-(2,3-dihydro-1,4-benzodioxin-5-yl)-4-pipéridyl]amino}-1-indanol et le (*trans*)-2-{[1-(2,3-dihydro-1,4-benzodioxin-5-yl)-4-pipéridyl]amino}-1-indanol, ainsi que leurs sels d'addition à un acide ou à une base pharmaceutiquement acceptable.

7. Composés de formule (I) selon la revendication 1 qui sont le (*cis*)-2-{[1-(3,4-dihydro-2*H*-chromén-8-yl)-4-pipéridyl]amino}-6-fluoro-1-indanol, et le *(trans)-2*-{[1-(3,4-dihydro-2*H*-chromén-8-yl)-4-pipéridyl]amino}-6-fluoro-1-indanol, ainsi que leurs sels d'addition à un acide ou à une base pharmaceutiquement acceptable.

8. Composés de formule (I) selon la revendication 1 qui sont le (*cis*)-2-{[1-(2,3-dihydro-1,4-benzodioxin-5-yl)-4-pipéridyl]amino}-6-méthyl-1-indanol, et le (*trans*)-2-{[1-(2,3-dihydro-1,4-benzodioxin-5-yl)-4-pipéridyl]amino}-6-méthyl-1-indanol, ainsi que leurs sels d'addition à un acide ou à une base pharmaceutiquement acceptable.

9. Composés de formule (I) selon la revendication 1 qui sont le (*cis*)-2-{[1-(3,4-dihydro-2*H*-chromén-8-yl)-4-pipéridyl]amino}-5,6-méthylènedioxy-1-indanol, et le (trans)-2-{[1-(3,4-dihydro-2*H*-chromén-8-yl)-4-pipéridyl]amino}-5,6-méthylènedioxy-1-indanol, ainsi que leurs sels d'addition à un acide ou à une base pharmaceutiquement acceptable.

10. Composés de formule (I) selon la revendication 1 qui sont le (*cis*)-2-{[1-(3,4-dihydro-2*H*-chromén-8-yl)-4-pipéridyl]amino}-5,6-dihydrofuro[2,3-b]indan-1-ol, et le (trans)-(2-{[1-(3,4-dihydro-2*H*-chromén-8-yl)-4-pipéridyl]amino}-5,6-dihydrofuro[2,3-b]indan-1-ol, ainsi que leurs sels d'addition à un acide ou à une base pharmaceutiquement acceptable.

11. Procédé de préparation des composés de formule (I) selon la revendication 1 **caractérisé en ce que** l'on utilise comme produit de départ, un composé de formule (II) : 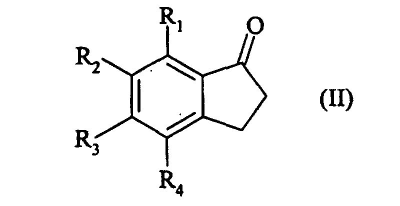 dans laquelle R₁, R₂, R₃ et R₄ ont la même signification que dans la formule (I),
composés de formule (II) que l'on fait réagir avec de l'isoamylnitrite, en présence d'acide chlorhydrique dans le méthanol, pour conduire aux composés de formule (III) : 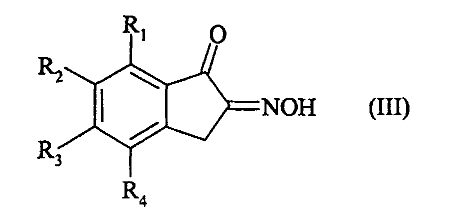 dans laquelle R₁, R₂, R₃ et R₄ sont tels que définis précédemment,
composés de formule (III) que l'on place en présence d'un agent de réduction, pour conduire aux composés de formule (IV), 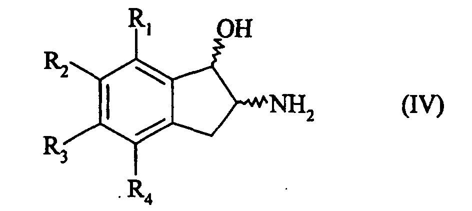 dans laquelle R₁, R₂, R₃ et R₄ sont tels que définis précédemment,
composés de formule (IV) que l'on traite, dans des conditions d'amination réductrice, par un composé de formule (V) : 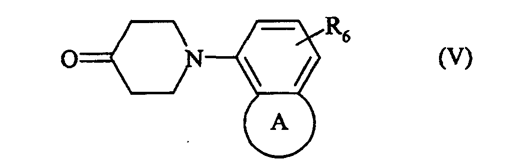 dans laquelle A et R₆ ont la même signification que dans la formule (I),
pour conduire aux composés de formule (I/a), cas particulier des composés de formule (I) : 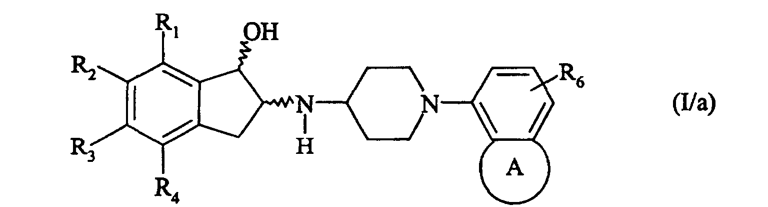 dans laquelle R₁, R₂, R₃, R₄, R₆ et A sont tels que définis précédemment,
composés de formule (I/a) que l'on sépare selon des méthodes classiques de séparation, en ces deux composants de formules (I/a *cis*) et (I/a trans), cas particuliers des composés de formule (I) : 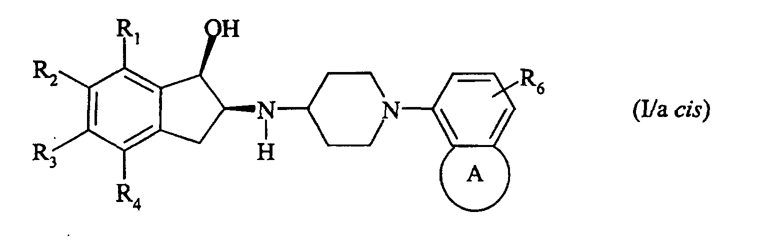 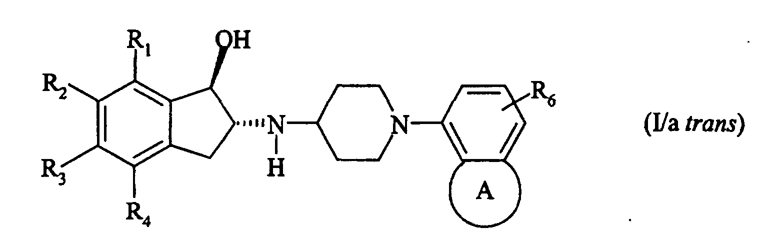 dans lesquelles R₁, R₂, R₃, R₄, R₆ et A sont tels que définis précédemment,
composés de formules (I/a), (I/a *cis*) et (I/a trans), dont on substitue, si on le désire, la fonction amine secondaire, selon des méthodes classiques de la chimie organique, en utilisant un composé de formule (VI) :
R'₅₋Z (IV)
dans laquelle R'₅ représente un groupement alkyle (C₁-C₆) linéaire ou ramifié ou arylalkyle (C₁-C₆) linéaire ou ramifié, et Z un groupement libérable,
pour conduire respectivement aux composés de formules (I/b), (I/b *cis*) et (I/b trans) : 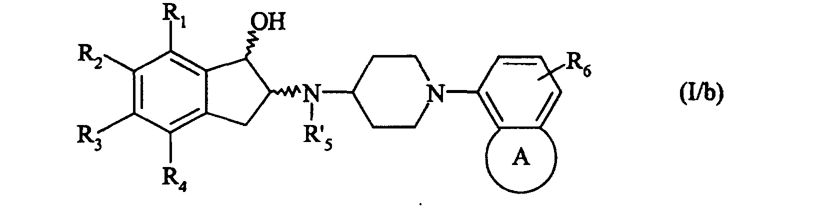 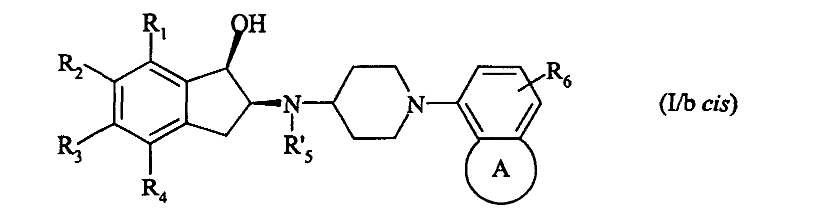 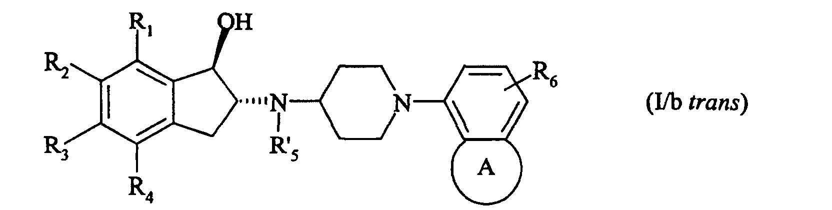 dans lesquelles R₁, R₂, R₃, R₄, R'₅, R₆ et A sont tels que définis précédemment,
les composés de formules (I/a), (I/b), (I/a *cis*), (I/a trans ), (I/b *cis*) et (I/b trans ), formant l'ensemble des composés de formule (I), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

12. Compositions pharmaceutiques contenant comme principe actif au moins un composé de formule (I) selon l'une quelconque des revendications 1 à 10, seul ou en combinaison avec un ou plusieurs excipients ou véhicules inertes, non toxiques, pharmaceutiquement acceptables.

13. Compositions pharmaceutiques selon la revendication 12 contenant au moins un principe actif selon l'une quelconque des revendications 1 à 10, utiles dans le traitement des pathologies dans lesquelles interviennent les récepteurs 5HT_{1B}, telles que la dépression, l'anxiété, les troubles impulsifs, les maladies psychiatriques liées à un disfonctionnement de la transmission sérotoninergique, les maladies dégénératives, la douleur, la migraine, les céphalés, les accidents vasculaires cérébraux, la boulimie, l'anorexie, les maladies cardio-vasculaires telles que l'angor instable.

## Patentansprüche

1. Verbindungen der Formel (I): 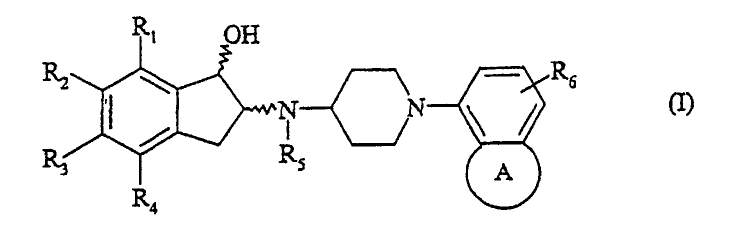 in der:
• R₁, R₂, R₃ und R₄, die gleichartig oder verschieden sind, jeweils unabhängig voneinander Wasserstoffatome, Halogenatome, geradkettige oder verzweigte (C₁-C₆)-Alkylgruppen, geradkettige oder verzweigte (C₂-C₆)-Alkenylgruppen, geradkettige oder verzweigte (C₂-C₆)-Alkinylgruppen, Hydroxygruppen, geradkettige oder verzweigte (C₁-C₆)-Alkoxygruppen, geradkettige oder verzweigte (C₁-C₆)-Trihalogenalkylgruppen bedeuten oder jeweils zwei Gruppen in benachbarter Position einen Heterocyclus bilden, der aus einer monocyclischen Gruppe mit 5 bis 7 Kettengliedern gebildet ist, welcher gegebenenfalls eine oder zwei zusätzliche Unsättigungen zu jener der Bindung des Heterocyclus mit dem Phenylkern, an den er gebunden ist, aufweist und ein, zwei oder drei gleichartige oder verschiedene Heteroatome ausgewählt aus Sauerstoff, Schwefel oder Stickstoff enthält,
• R₅ ein Wasserstoffatom, eine geradkettige oder verzweigte (C₁-C₆)-Alkylgruppe oder eine geradkettige oder verzweigte Aryl-(C₁-C₆)-alkylgruppe bedeutet,
• R₆ ein Wasserstoffatom, ein Halogenatom, eine geradkettige oder verzweigte (C₁-C₆)-Alkylgruppe, eine Hydroxygruppe, eine geradkettige oder verzweigte (C₁-C₆)-Alkoxygruppe oder eine geradkettige oder verzweigte (C₁-C₆)-Trihalogenalkylgruppe bedeutet,
• A zusammen mit den Kohlenstoffatomen des Phenylkerns, an den sie gebunden ist, eine Chromangruppe oder eine 2,3-Dihydro-1,4-benzodioxingruppe darstellt,
deren Isomere sowie deren Additionssalze mit einer pharmazeutisch annehmbaren Säure oder Base.

2. Verbindungen der Formel (I) nach Anspruch 1, **dadurch gekennzeichnet, daß** R₅ ein Wasserstoffatom bedeutet, deren Isomere sowie deren Additionssalze mit einer pharmazeutisch annehmbaren Säure oder Base.

3. Verbindungen der Formel (I) nach Anspruch 1, **dadurch gekennzeichnet, daß** R₁, R₂ und R₃, die gleichartig oder verschieden sind, unabhängig voneinander Wasserstoffatome, Halogenatome oder geradkettige oder verzweigte (C₁-C₆)-Alkylgruppen bedeuten oder jeweils zwei Gruppen in benachbarter Position mit den Kohlenstoffatomen des Phenylkerns, an den sie gebunden sind, einen Heterocyclus mit 5 Kettengliedern bilden, der ein oder zwei Sauerstoffatome aufweist, deren Isomere sowie deren Additionssalze mit einer pharmazeutisch annehmbaren Säure oder Base.

4. Verbindungen der Formel (I) nach Anspruch 1, **dadurch gekennzeichnet, daß** R₆ ein Wasserstoffatom oder ein Halogenatom bedeutet, deren Isomere sowie deren Additionssalze mit einer pharmazeutisch annehmbaren Säure oder Base.

5. Verbindungen der Formel (I) nach Anspruch 1, nämlich (*cis*)-2-{[1-(3,4-Dihydro-2*H*-chromen-8-yl)-4-piperidyl]-amino}-5-fluor-1-indanol und (*trans*)-2-{[1-(3,4-Dihydro-2*H*-chromen-8-yl)-4-piperidyll-amino}-5-fluor-1-indanol, sowie deren Additionssalze mit einer pharmazeutisch annehmbaren Säure oder Base.

6. Verbindungen der Formel (I) nach Anspruch 1, nämlich (*cis*)-2-{[1-(2,3-Dihydro-1,4-benzodioxin-5-yl)-4-piperidyl]-amino}-1-indanol und (*trans*)-2-{[1-(2,3-Dihydro-1,4-benzodioxin-5-yl)-4-piperidyl]-amino}-1-indanol, sowie deren Additionssalze mit einer pharmazeutisch annehmbaren Säure oder Base.

7. Verbindungen der Formel (I) nach Anspruch 1, nämlich (*cis*)-2-{[1-(3,4-Dihydro-2*H*-chromen-8-yl)-4-piperidyl]-amino}-6-fluor-1-indanol und (*trans*)-2-{[1-(3,4-Dihydro-2*H*-chromen-8-yl)-4-piperidyl]-amino}-6-fluor-1-indanol, sowie deren Additionssalze mit einer pharmazeutisch annehmbaren Säure oder Base.

8. Verbindungen der Formel (I) nach Anspruch 1, nämlich (*cis*)-2-{[1-(2,3-Dihydro-1,4-benzodioxin-5-yl)-4-piperidyl]-amino}-6-methyl-1-indanol und (*trans*)-2-{[1-(2,3-Dihydro-1,4-benzodioxin-5-yl)-4-piperidyl]-amino}-6-methyl-1-indanol, sowie deren Additionssalze mit einer pharmazeutisch annehmbaren Säure oder Base.

9. Verbindungen der Formel (I) nach Anspruch 1, nämlich (*cis*)-2-{[1-(3,4-Dihydro-2*H*-chromen-8-yl)-4-piperidyl]-amino}-5,6-methylendioxy-1-indanol und (*trans*)-2-{[1-(3,4-Dihydro-2*H*-chromen-8-yl)-4-piperidyl]-amino}-5,6-methylendioxy-1-indanol, sowie deren Additionssalze mit einer pharmazeutisch annehmbaren Säure oder Base.

10. Verbindungen der Formel (I) nach Anspruch 1, nämlich (*cis*)-2-{[1-(3,4-Dihydro-2*H*-chromen-8-yl)-4-piperidyl]-amino}-5,6-dihydrofuro[2,3-b]indan-1-ol und (*trans*)-2-{[1-(3,4-Dihydro-2*H*-chromen-8-yl)-4-piperidyl]-amino}-5,6-dihydrofuro-[2,3-b]indan-1-ol, sowie deren Additionssalze mit einer pharmazeutisch annehmbaren Säure oder Base.

11. Verfahren zur Herstellung der Verbindungen der Formel (I) nach Anspruch 1, **dadurch gekennzeichnet, daß** man als Ausgangsprodukt eine Verbindung der Formel (II) verwendet: 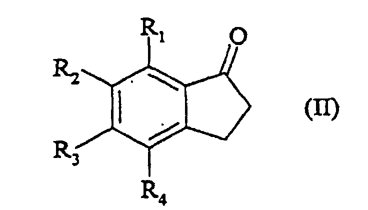 in der R₁, R₂, R₃ und R₄ die bezüglich der Formel (I) angegebenen Bedeutungen besitzen,
welche Verbindungen der Formel (II) man mit Isoamylnitrit in Gegenwart von Chlorwasserstoffsäure in Methanol umsetzt zur Bildung der Verbindungen der Formel (III): 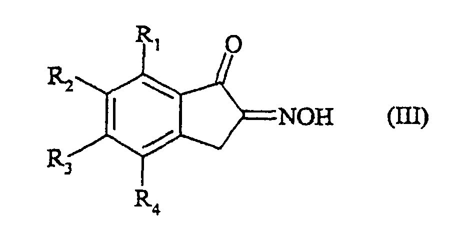 in der R1, R₂, R₃ und R₄ die oben angegebenen Bedeutungen besitzen,
welche Verbindungen der Formel (III) man mit einem Reduktionsmittel behandelt zur Bildung der Verbindungen der Formel (IV): 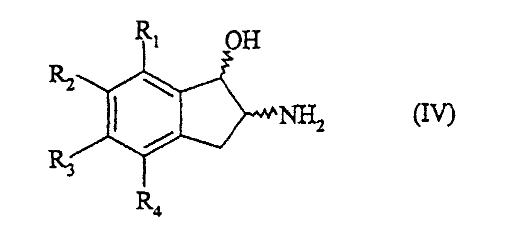 in der R₁, R₂, R₃ und R₄ die oben angegebenen Bedeutungen besitzen,
welche Verbindungen der Formel (IV) man unter den Bedingungen der reduktiven Aminierung mit einer Verbindung der Formel (V) behandelt: 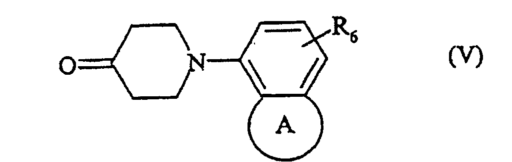 in der A und R₆ die bezüglich der Formel (I) angegebenen Bedeutungen besitzen,
zur Bildung der Verbindungen der Formel (I/a), einem Sonderfall der Verbindungen der Formel (I): 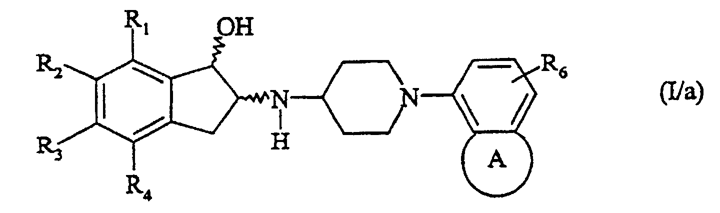 in der R₁, R₂, R₃, R₄, R₆ und A die oben angegebenen Bedeutungen besitzen,
welche Verbindungen der Formel (I/a) man mit Hilfe klassischer Trennungsmethoden in die beiden Komponenten der Formeln (I/a *cis*) und (I/a *trans*) trennt, Sonderfällen der Verbindungen der Formel (I): 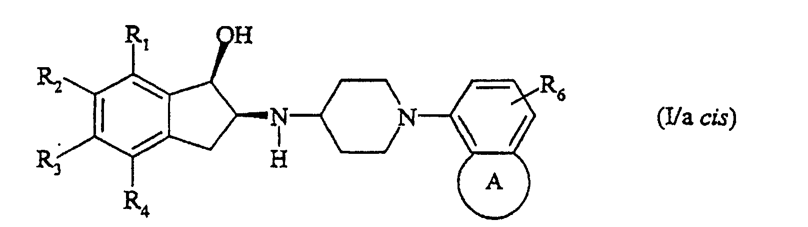 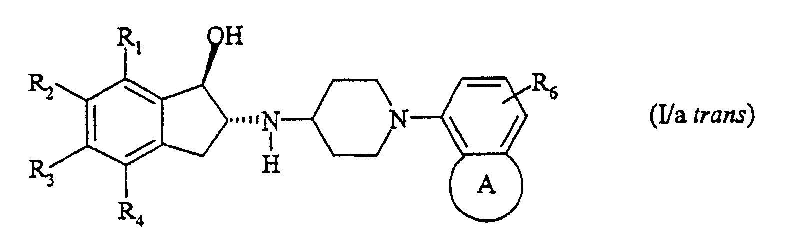 worin R₁, R₂, R₃, R₄, R₆ und A die oben angegebenen Bedeutungen besitzen,
bei welchen Verbindungen der Formeln (I/a), (I/a *cis*) und (I/a *trans*) man gewünschtenfalls die sekundäre Aminfunktion mit Hilfe klassischer Methoden der organischen Chemie unter Verwendung einer Verbindung der Formel (VI):
R'₅ - Z (VI)
in der R'₅ eine geradkettige oder verzweigte (C₁-C₆)-Mkylgruppe oder eine geradkettige oder verzweigte Aryl-(C₁-C₆)-alkylgruppe und Z eine freisetzbare Gruppe bedeuten, substituiert
zur Bildung der entsprechenden Verbindungen der Formeln (I/b), (I/b *cis*) und (I/b *trans*): 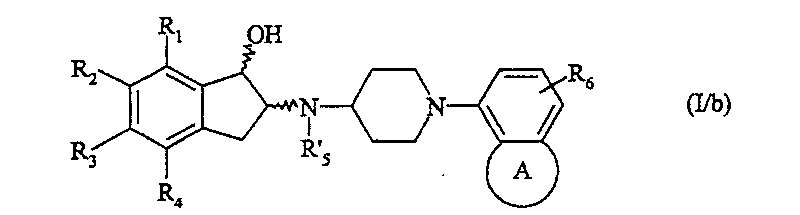 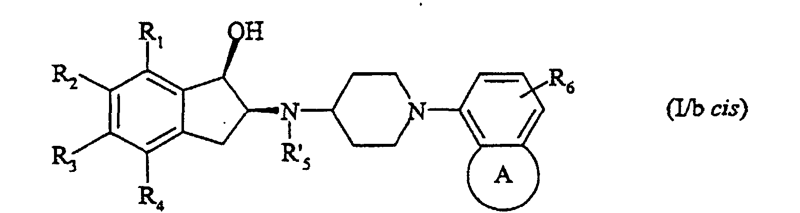 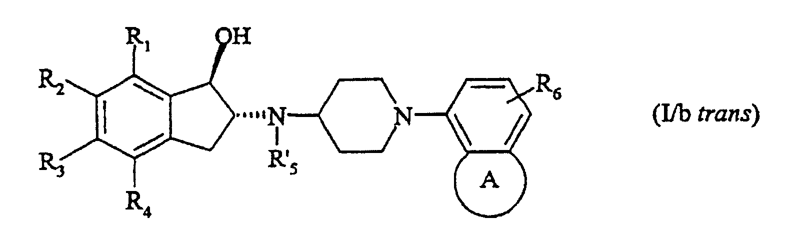 in denen R₁, R₂, R₃, R₄, R'₅, R₆ und A die oben angegebenen Bedeutungen besitzen,
wobei die Verbindungen der Formeln (I/a), (I/b), (I/a *cis*), (I/a *trans*), (I/b *cis*) und (I/b *trans*) die Gesamtheit der Verbindungen der Formel (I) bilden, welche man gegebenenfalls mit Hilfe einer klassischen Reinigungsmethode reinigt, gegebenenfalls mit Hilfe einer klassischen Trennungsmethode in die Isomeren auftrennt und gewünschtenfalls in ihre Additionssalze mit einer pharmazeutisch annehmbaren Säure oder Base umwandelt.

12. Pharmazeutische Zubereitungen enthaltend als Wirkstoff mindestens eine Verbindung der Formel (I) nach einem der Ansprüche 1 bis 10 allein oder in Kombination mit einem oder mehreren inerten, nichttoxischen, pharmazeutisch annehmbaren Trägermaterialien oder Bindemitteln.

13. Pharmazeutische Zubereitungen nach Anspruch 12 enthaltend mindestens einen Wirkstoff nach einem der Ansprüche 1 bis 10 für die Behandlung von pathologischen Zuständen, bei denen die Rezeptoren 5HT_{1B} beteiligt sind, wie der Depression, der Angst, von impulsiven Störungen, von psychiatrischen Erkrankungen, die mit einer Dysfunktion der serotoninergischen Transmission verbunden sind, von degenerativen Erkrankungen, von Schmerzen, der Migräne, Kopfschmerzen, Zerebralgefäßvorfällen, der Bulimie, der Anorexie und von kardiovaskulären Erkrankungen, wie instabilem Angor.

## Claims

1. Compounds of formula (I): 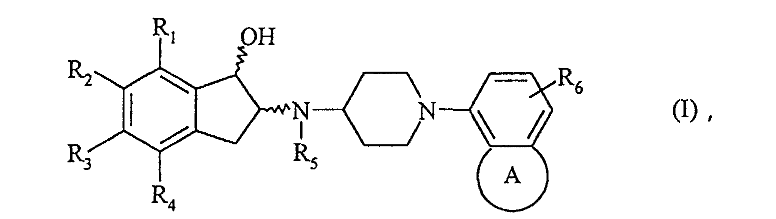 wherein :
• R₁, R₂, R₃ and R₄, which may be identical or different, each represents independently of the others a hydrogen atom, a halogen atom, a linear or branched (C₁-C₆)alkyl group, a linear or branched (C₂-C₆)alkenyl group, a linear or branched (C₂-C₆)alkynyl group, a hydroxy group, a linear or branched (C₁-C₆)alkoxy group or a linear or branched (C₁-C₆)-trihaloalkyl group, or any pair in adjacent positions may form a heterocycle composed of a monocyclic, 5- to 7-membered group optionally containing one or two unsaturations additional to that due to the linkage of the said heterocycle to the phenyl nucleus with which it is fused and containing one, two or three identical or different hetero atoms selected from oxygen, sulphur and nitrogen,
• R₅ represents a hydrogen atom, a linear or branched (C₁-C₆)alkyl group or an aryl-(C₁-C₆)alkyl group in which the alkyl moiety may be linear or branched,
• R₆ represents a hydrogen atom, a halogen atom, a linear or branched (C₁-C₆)alkyl group, a hydroxy group, a linear or branched (C₁-C₆)alkoxy group or a linear or branched (C₁-C₆)trihaloalkyl group, and
• A, together with the carbon atoms of the phenyl nucleus to which it is bonded, represents a chroman or 2,3-dihydro-1,4-benzodioxin group,
isomers thereof and addition salts thereof with a pharmaceutically acceptable acid or base.

2. Compounds of formula (I) according to claim 1, **characterised in that** R₅ represents a hydrogen atom, isomers thereof and addition salts thereof with a pharmaceutically acceptable acid or base.

3. Compounds of formula (I) according to claim 1, **characterised in that** R₁, R₂, R₃ and R₄, which may be identical or different, each represents independently of the others a hydrogen atom, a halogen atom or a linear or branched (C₁-C₆)alkyl group, or any pair in adjacent positions may form, together with the carbon atoms of the phenyl nucleus to which they are bonded, a 5-membered heterocycle containing one or two oxygen atoms, isomers thereof and addition salts thereof with a pharmaceutically acceptable acid or base.

4. Compounds of formula (I) according to claim 1, **characterised in that** R₆ represents a hydrogen atom or a halogen atom, isomers thereof and addition salts thereof with a pharmaceutically acceptable acid or base.

5. Compounds of formula (I) according to claim 1 which are (*cis*)-2-{[1-(3,4-dihydro-2*H*-chromen-8-yl)-4-piperidyl]amino}-5-fluoro-1-indanol and (*trans*)-2-{[1*-*(3,4-dihydro-2*H*-chromen-8-yl)-4-piperidyl]amino}-5-fluoro-1-indanol, and addition salts thereof with a pharmaceutically acceptable acid or base.

6. Compounds of formula (I) according to claim 1 which are (*cis*)-2-{[1-(2,3-dihydro-1,4-benzodioxin-5-yl)-4-piperidyl]amino}-1-indanol and (*trans*)-2-{[1-(2,3-dihydro-1,4-benzodioxin-5-yl)-4-piperidyl]amino}-1-indanol, and addition salts thereof with a pharmaceutically acceptable acid or base.

7. Compounds of formula (I) according to claim 1 which are (*cis*)-2-{[1-(3,4-dihydro-2*H*-chromen-8-yl)-4-piperidyl]amino}-6-fluoro-1-indanol and (*trans*)-2-{[1-(3,4-dihydro-2*H*-chromen-8-yl)-4-piperidyl]amino}-6-fluoro-1-indanol, and addition salts thereof with a pharmaceutically acceptable acid or base.

8. Compounds of formula (I) according to claim 1 which are (*cis*)-2-{[1-(2,3-dihydro-1,4-benzodioxin-5-yl)-4-piperidyl]amino}-6-methyl-1-indanol and (*trans*)-2-{[1-(2,3-dihydro-1,4-benzodioxin-5-yl)-4-piperidyl]amino}-6-methyl-1-indanol, and addition salts thereof with a pharmaceutically acceptable acid or base.

9. Compounds of formula (I) according to claim 1 which are (*cis*)-2-{[1-(3,4-dihydro-2*H*-chromen-8-yl)-4-piperidyl]amino}-5,6-methylenedioxy-1-indanol and (*trans*)-2-{[1-(3,4-dihydro-2*H*-chromen-8-yl)-4-piperidyl]amino}-5,6-methylenedioxy-1-indanol, and addition salts thereof with a pharmaceutically acceptable acid or base.

10. Compounds of formula (I) according to claim 1 which are (*cis*)-2-{[1-(3,4-dihydro-2*H*-chromen-8-yl)-4-piperidyl]amino}-5,6-dihydrofuro[2,3-b]indan-1-ol and (*trans*)-(2-{[1-(3,4-dihydro-2*H*-chromen-8-yl)-4-piperidyl]amino}-5,6-dihydrofuro[2,3-b]indan-1-ol, and addition salts thereof with a pharmaceutically acceptable acid or base.

11. Process for the preparation of compounds of formula (I) according to claim 1, **characterised in that** there is used as starting material a compound of formula (II): 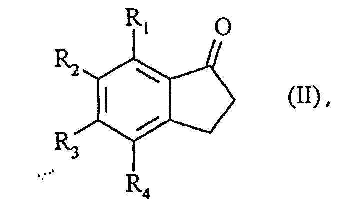 wherein R₁, R₂, R₃ and R₄ are as defined for formula (I),
which compounds of formula (II) are reacted with isoamyl nitrite, in the presence of hydrochloric acid in methanol, to yield the compounds of formula (III) : 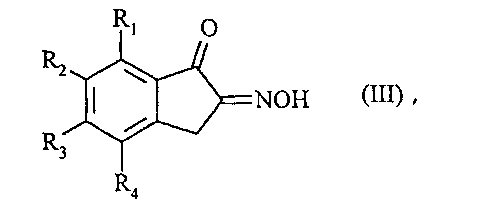 wherein R₁, R₂, R₃ and R₄ are as defined hereinbefore,
which compounds of formula (III) are placed in the presence of a reducing agent to yield the compounds of formula (IV) : 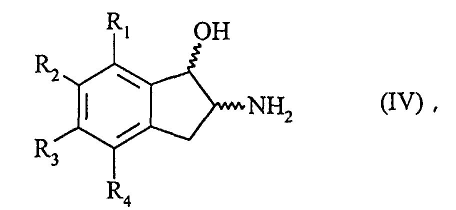 wherein R₁, R₂, R₃ and R₄ are as defined hereinbefore,
which compounds of formula (IV) are treated, under conditions of reductive amination, with a compound of formula (V) : 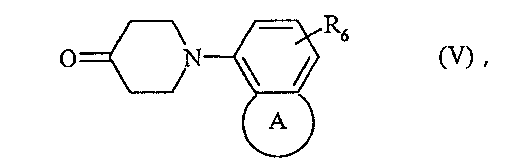 wherein A and R₆ are as defined for formula (I),
to yield the compounds of formula (I/a), a particular case of the compounds of formula (I) : 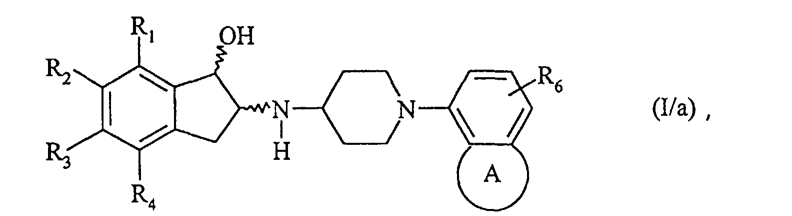 wherein R₁, R₂, R₃, R₄, R₆ and A are as defined hereinbefore,
which compounds of formula (I/a) are separated according to conventional separation techniques into the following two compounds of formulae (I/a *cis)* and (I/a *trans*), particular cases of the compounds of formula (I) : 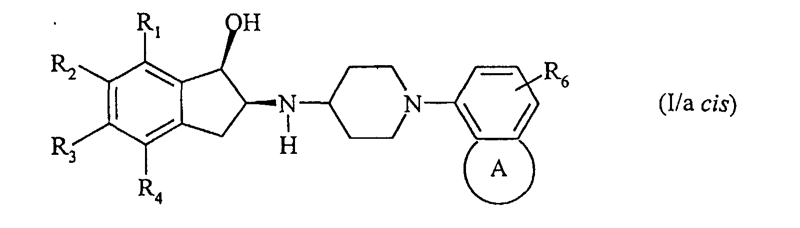 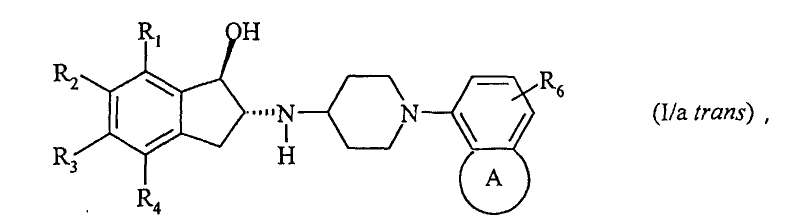 wherein R₁, R₂, R₃, R₄, R₆ and A are as defined hereinbefore,
the secondary amine function of which compounds of formulae (I/a), (I/a *cis*) and (I/a *trans*) is substituted, if desired, according to conventional techniques of organic chemistry using a compound of formula (VI) :
R'₅-Z (VI),
wherein R'₅ represents a linear or branched (C₁-C₆)alkyl group or an aryl-(C₁-C₆)alkyl group in which the alkyl moiety may be linear or branched, and Z represents a leaving group,
to yield the compounds of formulae (I/b), (I/b *cis)* and (I/b *trans*), respectively : 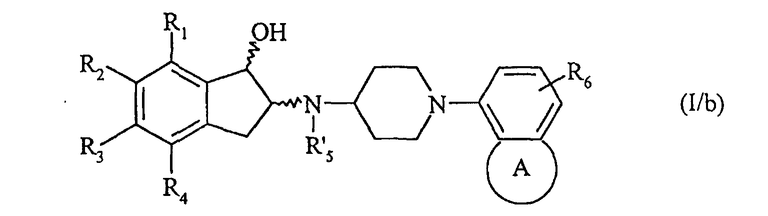 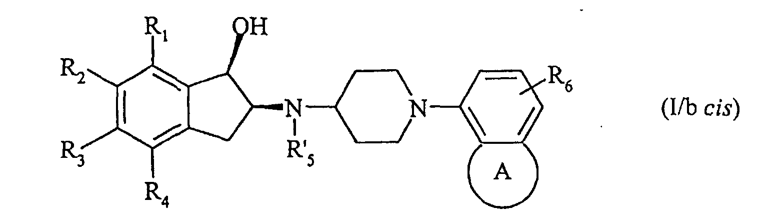 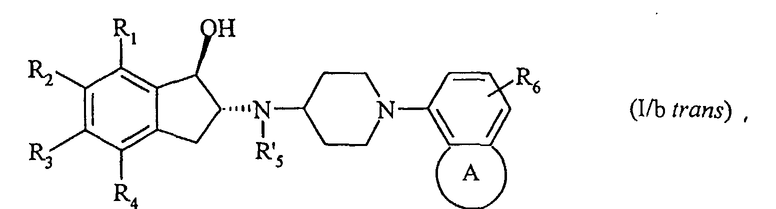 wherein R₁, R₂, R₃, R₄, R'₅, R₆ and A are as defined hereinbefore,
which compounds of formulae (I/a), (I/b), (I/a *cis*), (I/a *trans*), (I/b *cis*) and (I/b *trans*), which constitute the totality of the compounds of formula (I), are, if necessary, purified according to a conventional purification technique, are optionally separated into their isomers according to a conventional separation technique and, if desired, are converted into their addition salts with a pharmaceutically acceptable acid or base.

12. Pharmaceutical compositions comprising as active ingredient at least one compound of formula (I) according to any one of claims 1 to 10, alone or in combination with one or more pharmaceutically acceptable, inert, non-toxic excipients or carriers.

13. Pharmaceutical compositions according to claim 12 comprising at least one active ingredient according to any one of claims 1 to 10 for use in the treatment of pathologies in which 5HT_{1B} receptors are involved, such as depression, anxiety, impulsive disorders, psychiatric disorders associated with dysfunction of serotoninergic transmission, degenerative diseases, pain, migraine, headaches, cerebral vascular accidents, bulimia, anorexia and cardiovascular disorders, such as unstable angina.
